# FASCICULE DE BREVET EUROPEEN

(11) **EP 0 499 555 B1**
(45) Date de publication et mention de la délivrance du brevet: **17.05.2000**
(21) Numéro de dépôt: 92430003.1
(22) Date de dépôt: 12.02.1992
(51) Int. Cl.: C12N 15/12, C12N 15/79, C12P 21/08, A61K 39/395, G01N 33/567

(54) **Molécules d'ADN recombinants codant pour une chaîne du récepteur de l'antigène des cellules T, procédé de préparation, anticorps et médicaments les renfermant**
Rekombinante DNS die für eine Kette des T-Zell Rezeptors kodiert, Verfahren zur Herstellung, Antikörper und Arzneimittel die diese enthalten
Recombinant DNA coding for a T-cell receptor chain, process for preparation and antibodies and pharmaceutical compositions comprising the same

(30) Priorité: 15.02.1991 FR 9102189
(43) Date de publication de la demande: 19.08.1992
(73) Titulaire: IMMUNOTECH S.A., F-13276 Marseille Cédex 9 (FR)
(72) Inventeur: Romagne, François, F-13008 Marseille (FR); van Agthoven, André, F-13009 Marseille (FR); Malissen, Bernard, F-13009 Marseille (FR)
(74) Mandataire: Rinuy, Santarelli

(56) Documents cités:
- EP-A- 0 340 793
- EP-A- 0 403 156
- CELL. vol. 60, no. 6, 23 Mars 1990, CAMBRIDGE, NA US pages 929 - 939; GOVERMAN, J. ET AL.: 'Chimeric Immunoglobulin T-cell receptor proteins from functional receptors' activation. '
- JOURNAL OF BIOLOGICAL CHEMISTRY. vol. 264, no. 13, 5 Mai 1989, BALTIMORE US pages 7310 - 7316; MARIUZZA, R.A. ET AL.: 'Scecretion of heterodimeric Valpha and Ck T-cell receptor-Immunoglobulin'

## Description

La présente invention concerne de nouvelles molécules d' ADN recombinants codant pour une chaîne du récepteur pour l'antigène des cellules T, procédé de préparation, anticorps et médicaments les renfermant.

Il serait intéressant de développer facilement un panel d'anticorps dirigé contre les différentes chaines de récepteurs pour l'antigène des cellules T (ci-après TCR) humaines, afin par exemple d'obtenir une discrimination et une numération précise entre les différentes sous-populations de cellules T humaines.

L'étude du déséquilibre entre les sous-populations permettrait le diagnostic différentiel des maladies provoquant une prolifération de cellules T. Elle pourrait aussi être le témoin d'une réponse immunitaire spécifique contre un tissu particulier, pathogène tel qu'une tumeur par exemple. On sait en particulier que des anticorps contre les antigènes CD₄ et CD₈ qui distinguent deux populations de cellules T, sont témoins d'une série de maladies immunitaires dont le SIDA.

Ces anticorps devraient pouvoir être réalisés facilement et en grande quantité.

EP-A-O 340 793 décrit des ADN recombinants chimériques dont les deux segments recombinants proviennent ou non de deux espèces différentes. Mais les chimères obtenues à partir de ces deux segments codent pour des domaines immunoglobulines, l'un variable codant pour un segment d'immunoglobuline, l'autre, constant, pour un segment de TCR.

CHOI, Y et al, a décrit dans Nature 346 - pages 471-473 (1990) des ADN codant pour des TCR chimères, comprenant un segment Vβ humain, ligué à un segment Dβ, Jβ, Cβ2 murin. Le TCR chimère ainsi formé, couplé à une chaîne α et associé au CD3 a été exprimé. Il est suggéré que le procédé décrit peut permettre la production d'anticorps de souris spécifiques des segments Vβ ou Vα du TCR humain. Toutefois, ce procédé décrit est spécifique d'un type de construction donnée contenant la partie Vβ humaine seulement. Il serait intéressant de pouvoir utiliser un segment Cβ2 d'origine murine pour permettre la préparation d'une large série de chimères comprenant différentes parties V, J et D, notamment humaines.

La demanderesse a découvert de nouveaux ADN recombinants permettant la préparation de nouveaux vecteurs d'expression eucaryote qui étaient de remarquables agents de transfection de cellules eucaryotes, lesquelles cellules transfectées étaient d'excellents immunogènes conduisant à des anticorps, notamment monoclonaux, dirigés vers les différentes chaines des TCR humains.

C'est pourquoi la présente demande a pour objet un ADN recombinant caractérisé en ce qu'il comprend une séquence nucléotidique chimère codant pour une chaîne du récepteur pour l'antigène des cellules T, ladite séquence étant constituée :
- d'une séquence provenant d'un mammifère autre que murin et codant pour la totalité de la partie variable (V), diversité (D) et de jonction (J),
- et d'une séquence d'origine murine codant pour la partie complémentaire constante (C) absente de la séquence de mammifère ci-dessus,
les deux séquences étant liées au niveau d'un site de restriction naturel ou créé artificiellement. Dans ce qui suit, on appellera ce site "site sélectionné".

Par ADN recombinant l'on entend une séquence de nucléotides artificiellement crée.

Par chimère l'on entend que l'ADN recombinant comprend au moins deux séquences provenant d'origines différentes,
par exemple 3, 4 ou 5 et de préférence 2 séquences de deux origines différentes.

Ces séquences proviennent de deux espèces différentes et l'origine de l'une d'entre elles est murine ; l'autre (ou les autres) proviendra notamment d'un primate et de préférence de l'homme.

Le récepteur des cellules T, est une protéine hétérodimère composée de deux chaînes polypeptidiques reliées par un pont disulfure. Le récepteur est exprimé à la surface des cellules T en association avec le complexe CD3. Le locus correspondant à une chaîne particulière du TCR comprend une importante sélection de segments géniques distincts, incluant de nombreux segments V différents, plusieurs segments J et un ou deux segments constants C. Ces segments peuvent subir des réarrangements somatiques pendant la maturation thymique des cellules T produisant ainsi un gène unique, qui est transcrit et contenant des segments V, des segments J, un segment C et, le cas échéant, un segment D entre les segments V et J.

Dans l'ADN recombinant chimère, ci-dessus décrit, la totalité de la séquence codant pour la partie C peut provenir d'un murin et dans ce cas la séquence de l'autre mammifère ne comprend aucun nucléotide correspondant à la partie C. De préférence, la séquence provenant d'un autre mammifère comprend une courte partie C, de préférence inférieure à 100 nucléotides et notamment environ 60 nucléotides ; de ce fait, seule une large partie C de la chimère provient du murin (cette partie étant la partie complémentaire absente de la séquence de l'autre mammifère ci-dessus) et le reste provenant du mammifère. Les deux séquences d'origines différentes sont liées au niveau d'un site de restriction sélectionné, en phase dand le cadre de lecture.

La séquence de l'autre mammifère comprendra nécessairement en plus des parties V, J et éventuellement D et une partie de C, la totalité de la séquence de tête.

Le site de restriction sélectionné peut être naturellement présent sur chacune des deux séquences ou présent naturellement sur une seule des deux séquences, ledit site de restriction étant artificiellement créé sur l'autre séquence.Le site de restriction ci-dessus doit être unique sur le segment Cβ murin et de préférence unique sur le segment de l'autre mammifère.

Lorsque le site de restriction sélectionné est créé artificiellement, il peut être réalisé par exemple par déletion ou adjonction de nucléotides ou de préférence par mutation selon les méthodes connues en elles-mêmes, notamment par mutagénèse dirigée. L'altération ainsi réalisée de l'ADN permet l'introduction d'un site de restriction normalement absent. En particulier, un site BglII, présent naturellement au niveau de l'ADN codant pour les régions Cβ1 et Cβ2 humain, mais absent du gène codant pour la chaîne du TCR de la souris, peut être introduit dans le géne Cβ2 de la souris par mutagénèse dirigée, mais éventuellement mutagénèse par polymérisation en chaîne (PCR). La mutation permet de conserver le maximum d'homologie avec la ou les séquences de départ. Le site de restriction ainsi créé sera de préférence placé au niveau d'un site homologue au site au niveau duquel le site de restriction est naturellement présent chez le mammifère considéré, particulièrement l'homme, de manière à permettre la coupure, tant de la séquence murine que de la séquence de mammifère à l'aide de ladite enzyme, pour ensuite, par ligation en phase dans le cadre de lecture, créer la chimère désirée. De ce qui précède, on comprend que si l'on choisit une enzyme de restriction donnée, un seul site de restriction correspondant doit au plus être présent sur le segment Cβ murin et de préférence unique sur le segment de l'autre mammifère et de plus, au niveau de la séquence codant pour la partie C, à un emplacement permettant la transcription en phase d'un ADN codant notamment la chaîne du TCR. Dans un cas particulièrement intéressant, on peut produire tout une série de gènes codant pour une partie C d'un TCR murin, ligués à de nombreuses parties V différentes de TCR, notamment humain.

Des ADN recombinants préférés selon l'invention sont caractérisés en ce que le site de restriction est naturel sur la séquence codant pour la partie C du mammifère.

Des chimères tout particulièrement préférées selon l'invention ont une partie mammifère caractérisée en ce que sa séquence provenant d'un mammifère est la séquence codant pour des segments Vβ, Dβ, Jβ humains, et une courte partie Cβ1 ou Cβ2 jusqu'au site unique BglII de Cβ1 et Cβ2.

La partie murine des chimères notables selon l'invention est caractérisée en ce que le site de restriction, notamment BglII est artificiellement créé sur la chaîne d'origine murine.

L'ADN recombinant selon l'invention peut se présenter sous forme d'une séquence nucléotidique mono ou simple brin, limitée aux parties V, D, J et C, ci-dessus, ou comportant d'autres nucléotides, par exemple et notamment ceux constituant la séquence de tête.

C'est ainsi que la séquence ci-dessus peut en particulier être incluse dans un vecteur d'expression, et dans ce cas de préférence dans un vecteur d'expression eucaryote.

La séquence ci-dessus peut également être incluse en vue de sa réplication dans un autre vecteur, par exemple dans un plasmide tel que par exemple BLUESCRIPT. Comme vecteur d'expression eucaryote on peut citer tout particulièrement pHβPr1-Néo. On peut citer également pHβPᵣ1gpt et généralement tout vecteur d'expression eucaryote possédant les signaux de régulation promoteur et un signal de polyadénylation permettant l'expression de l'ADNc. Bien évidemment, le choix du couple vecteur-hôte est de la compétence de l'homme de l'art.

Des ADN recombinants tout particulièrement préférés selon l'invention sont caractérisés en ce qu'ils se présentent sous forme d'un vecteur d'expression eucaryote, lequel étant de préférence dérivé de pHβPr1-Néo.

L'ADN recombinant ci-dessus, notamment sous forme de vecteur d'expression eucaryote, peut être utilisé pour transfecter dans un hôte approprié la séquence codant pour le TCR. Des cellules hôtes ainsi transfectées, éventuellement traitées, par exemple préalablement transfectées avec la chaîne CD3 Zeta pour obtenir une meilleure expression, peuvent être utilisées comme immunogène de préférence chez la souris.

On peut ainsi classiquement préparer des anticorps, notamment monoclonaux, le criblage des hybridomes obtenus à partir de cellules de myélome et de cellules de rate du murin utilisé, pouvant se faire par la reconnaissance de la lignée transfectée, versus la non-reconnaissance de la lignée de départ.

C'est pourquoi, la présente demande a également pour objet les anticorps, notamment monoclonaux, caractérisés en ce qu'ils sont préparés par mise en oeuvre d'un ADN recombinant ci-dessus décrit.

Les anticorps ci-dessus présentent de remarquables propriétés car ils sont parfaitement caractérisés vis à vis des chaînes de TCR, et particulièrement vis à vis des régions variables V des chaînes de TCR, mais aussi vis-à-vis des régions D et J dans leur réarrangement typique pour un TCR donné.

Ces anticorps sont donc capables de distinguer la population des cellules T humaines et ainsi spécifiquement reconnaître, par exemple une maladie ou une déficience donnée.

Dans les anticorps ci-dessus décrits on préfère les anticorps anti partie variable, notamment Vβ, D et J qui présentent de remarquables propriétés à la fois pour le diagnostic et la thérapie, curative ou préventive, chez l'homme ou l'animal, des maladies auto-immunes.

C'est pourquoi la présente invention a aussi pour objet les compositions pharmaceutiques caractérisées en ce qu'elles renferment au moins un des anticorps tels que définis ci-dessus.

Les compositions pharmaceutiques peuvent se présenter notamment sous les formes parentérales, notamment injectables, couramment utilisées en médecine humaine, par exemple pour la voie sous cutanée, intramusculaire ou intraveineuse.

Les anticorps ci-dessus peuvent également être utilisés pour soigner les maladies immunes ou provoquant une prolifération des cellules T, ainsi que les leucémies à cellules T par injection des anticorps ci-dessus.

A cet effet, les anticorps peuvent être couplés à des toxines ou à des produits radioactifs, selon des méthodes connues en elles-mêmes.

Les anticorps selon l'invention trouvent leur emploi notamment dans le traitement des maladies immunes telles que l'arthrite rhumatoïde, le diabète, la maladie de Sjögren ou le lupus erythémateux.

La dose usuelle, variable selon le produit utilisé, le sujet traité et l'affection en cause, peut être par exemple de 0,001 à 25 mg par jour et par kg de poids corporel chez l'homme d'un anticorps monoclonal selon l'invention couplé à la ricine A à titre de cytotoxique, et de préférence de 0,05 à 0,2 mg/kg/jour.

Etant donné que des anticorps, notamment monoclonaux, ci-dessus trouvent aussi leur utilisation à des fins diagnostiques, pour la détection, l'identification ou le dosage des TCR ou de parties de TCR, la présente invention a également pour objet l'utilisation desdits anticorps ainsi que les compositions diagnostiques destinées à la détection, l'identification ou le dosage des antigènes ci-dessus.

Les anticorps ci-dessus trouvent également leur utilisation dans la purification des produits renfermant la partie antigénique correspondante à des chaînes de TCR.

Les anticorps ci-dessus peuvent être utilisés à fins diagnostiques en cytofluorimétrie et dans ce but peuvent être couplés, par des méthodes connues en elles-mêmes, à des marqueurs fluorescents tels que le FITC ou la phycoérythrine.

On peut, par exemple, détecter la présence de population de cellules T en détectant un segment variable spécifique au niveau des tissus souffrant par exemple d'une infection, d'un cancer ou d'une maladie auto-immune, et en détectant sa présence selon des méthodes connues en elles-mêmes.

On peut également marquer les anticorps ci-dessus décrits, à l'aide d'enzymes. On peut citer par exemple, la glucose 6 phosphate deshydrogénase, l'acétylcholinéstérase, la glucoseoxydase ou la bétagalactosidase.

Les anticorps totaux ci-dessus peuvent également être couplés à un radiomarqueur tel que l'indium 111, pour utilisation dans l'imagerie des populations de cellules T comportant une région variable spécifique, au niveau des parties malades du corps humain.

La présente invention a également pour objet un procédé de préparation des ADN recombinants ci-dessus décrits, caractérisé en ce qu'on ligue :
- une séquence nucléotidique constituée d'au moins la totalité de la séquence codant pour les parties V, D et J d'une chaîne du récepteur de l'antigène des cellules T d'un mammifère autre que murin jusqu'au niveau d'un site de restriction, avec
- une séquence nucléotidique d'origine murine comprenant la partie complémentaire de la partie C absente de la séquence de chaîne de mammifère ci-dessus, à partir du même site de restriction que ci-dessus,
ledit site de restriction étant artificiellement créé sur l'une, l'autre ou les deux séquences, pour obtenir la chimère attendue, puis si désiré, ligue une séquence comprenant la totalité des parties V,D,J et C ci-dessus avec un vecteur d'expression eucaryote, puis si désiré, isole la nouvelle chimère obtenue.

Dans des conditions préférentielles, le procédé décrit ci-dessus est caractérisé en ce qu'en outre l'on transfecte une cellule eucaryote comportant tous les éléments permettant la synthèse du récepteur de l'antigène des cellules T, à l'exception de la chaîne homologue à la chaîne codée par la séquence chimère ci-dessus, utilise comme antigène les cellules ainsi transfectées chez la souche murine correspondante, fusionne des cellules de rate prélevées sur les murins auxquels a été administré l'antigène à des cellules de myélome pour préparer, selon les méthodes connues en elles-mêmes, des anticorps dirigés contre le produit de la séquence variable du mammifère.

Le procédé ci-dessus décrit peut notamment être réalisé comme suit :
la séquence nucléotidique codant pour les parties V, D et J d'une chaîne du TCR de mammifère et notamment un ADN double brin linéaire comportant la séquence codant pour les parties V, J, éventuellement D et éventuellement C en partie, d'une chaîne de TCR de mammifère, de même que son gène de tête, est obtenue par coupure à l'aide de deux enzymes de restriction différentes : l'enzyme correspondante au site de restriction sélectionné au niveau de la partie C, par exemple BglII et une autre enzyme clivant la séquence du côté opposé, après le gène de tête, par exemple EcoRI à partir d'une séquence d'ADN double brin linéaire comportant ladite séquence. L'ADN ainsi obtenu est ligué à un vecteur traité par les mêmes deux enzymes de restriction, ce vecteur comportant la séquence codant pour le segment C de la chaîne de TCR, et les mêmes sites de restriction que ci-dessus, par exemple BglII et EcoRI . On obtient ainsi le gène chimère attendu. Celui-ci n'est toutefois pas exprimable tel quel. C'est pourquoi d'une part le vecteur comportant la chimère est traité par deux enzymes de restriction coupant de part et d'autre du gène chimère codant pour le TCR chimère ; d'autre part, on traite par les mêmes deux enzymes de restriction un vecteur d'expression eucaryote. Par ligation du gène obtenu ci-dessus codant pour le TCR chimère avec ledit vecteur d'expression eucaryote, on obtient un nouveau vecteur chimère d'expression eucaryote. Ledit vecteur d'expression eucaryote doit comporter les éléments nécessaires pour assurer la transcription correcte du gène chimère codant pour le TCR. A cet égard, on peut citer par exemple comme vecteur pHβPr1-Néo, qui peut être scindé par BamHI et SalI, et comprend notamment le gène de résistance à la néomycine utilisé comme un marqueur de sélection, un gène promoteur eucaryote SV40, ainsi qu'un signal polyadenylation à proximité du site BamHI. Le nouveau vecteur chimère obtenu est avantageusement utilisé pour transfecter des cellules eucaryotes, de préférence des cellules T de souris, notamment des lignées cellulaires comportant tous les éléments pour l'expression du TCR à l'exception du gène apporté par le vecteur chimère. On peut citer par exemple, la lignée de souris DOIS 19 ou celle du nom de DS 23.27.4. De telles cellules permettent une sélection facile des cellules transfectées qui seules sont capables d' exprimer le TCR à la surface de la cellule.

Bien entendu, chaque ligation (ci-dessus et ci-après) est suivie de la transfection d'un hôte approprié et la sélection des cellules contenant la chimère désirée.

Les cellules transfectées peuvent être administrées à titre d'antigène de préférence à des souris, par exemple par injection intrapéritonéale. On peut ainsi prélever des cellules de rate des murins ainsi traitées, cellules capables de secréter les anticorps recherchés pour les fusionner à des cellules immortelles, telles que le myélome. Les hybridomes sélectionnés produisant les anticorps recherchés permettent ainsi la préparation d'anticorps notamment monoclonaux dirigés contre une chaîne V particulière du TCR du mammifère choisi, notamment l'homme.

L'ADN double brin codant pour la chaîne du récepteur de l'antigène des cellules T de mammifère peut par exemple être préparé comme suit :
L'étude de la séquence des gènes codant pour une chaîne donnée permet de sélectionner un site de restriction déjà présent sur une partie constante C, soit alors amène à créer sur ladite séquence un tel site. La connaissance de ces séquences, par exemple en ce qui concerne les chaînes pour les familles Vβ1, Vβ2, Vβ3, Vβ4, Vβ5, Vβ6, Vβ7, Vβ8, Vβ10, Vβ11, Vβ12, Vβ15, Vβ17, Vβ18 permet la synthèse de nouveaux oligonucléotides utilisables pour synthétiser l'ADN complémentaire à partir de l'ARN messager.

On purifie à partir de lymphocytes T de mammifère (par exemple humains) l'ARNm codant pour une chaîne TCR donnée. On synthétise l'ADN correspondant à l'aide d'un oligonucléotide OHCβ spécifique d'un gène de mammifère donné et contenant le cas échéant le site unique de restriction sélectionné, synthétise le deuxième brin à partir d'un oligonucléotide spécifique de la chaîne et à l'aide de transcriptase reverse, et amplifie le gène obtenu par réactions de polymérisation en chaîne (PCR) en effectuant par exemple une trentaine de cycles.

On obtient ainsi une grande quantité d'ADN double brin linéaire codant. On peut alors couper par les enzymes appropriées, par exemple BglII et EcoRI, les fragments produits par PCR, de manière à éliminer tout ou partie de la séquence codant pour la partie C, à partir du site sélectionné et de manière à conserver les séquences V, D et J, ainsi que la totalité du gène de tête.
- La séquence nucléotidique d'origine murine comprenant la partie complémentaire de la partie C absente de la séquence de chaîne de mammifère ci-dessus, dans le cas où celle-ci se trouve sous la forme d'un vecteur comportant ladite séquence, peut être préparée comme suit :
   on isole un ADNc complet contenant toute la partie codante de la chaîne du TCR choisi à partir d'une banque d'ADNc provenant d'une lignée cellulaire murine, par exemple KB5C20, à l'aide d'une sonde connue ; on clone cet ADNc dans un plasmide approprié, par exemple PUC19, puis on transfecte le plasmide résultant appelé dans ce cas pUCmCβ2 dans un hôte, par exemple la souche bactérienne DH1 de E. coli.

On pourra ensuite cloner le fragment intéressant d'ADN murin dans un phage. Pour cela, on peut digérer pUCmCβ2 par des enzymes de restriction appropriées, par exemple, EcoRI et HindIII, de manière à obtenir trois fragments dans le cas ci-dessus. On purifie le fragment contenant toute la séquence codante de la chaîne C murine désirée (dans le cas des enzymes précitées, EcoRI coupe dans la partie V de la séquence). On obtient ainsi une séquence codant pour une partie de la partie V, ainsi que la totalité des parties D, J et C. On ligue alors ladite séquence avec un phage approprié, par exemple M13mp18, avec les mêmes enzymes de restriction. Dans ce cas, l'ADN phagique existe sous deux formes : simple brin pour le phage encapsidé et double brin pour sa forme réplicative. On hybride un oligonucléotide complémentaire de la région à muter, sauf pour la mutation désirée, avec la forme simple brin du phage. Le brin complémentaire est synthétisé in vitro par un ADN polymérase utilisant le même oligonucléotide pour initier la réaction. On ferme le nouveau brin sur lui-même à l'aide d'une ligase. Le double brin complètement homologue est alors transfecté par exemple dans E. coli, notamment JM101. Les colonies mutantes sont sélectionnées, par exemple par hybridation avec un oligonucléotide radio-marqué.

Pour des détails sur la mutagénèse dirigée, se référer à MANIATIS et al ( Molecular Cloning ; A laboratory Manual, 1989, Cold Spring Harbour Laboratory Press).

On purifie l'ADN double brin d'un sous-clone comportant la mutation désirée et dont le reste de la séquence codant pour la partie C est resté intègre, et le coupe par des enzymes appropriées, par exemple EcoRI et EcoRV pour isoler le fragment renfermant le gène désiré. On ligue alors ce fragment dans un plasmide adapté, qu'il soit possible de séquencer et comportant des sites de restriction permettant d'extraire facilement la séquence insérée pour l'introduire dans un vecteur d'expression et préalablement ouvert par des enzymes compatibles présentes dans son site de liaisons multiples, par exemple par EcoRI et SmaI. Comme à l'accoutumée,le produit de la ligation peut alors être transfecté dans un hôte approprié, par exemple une souche bactérienne E. coli DH1. En analysant, par exemple, par coupure avec deux couples d'enzymes tels que EcoRI-XBaI et EcoRI-BglII, on peut sélectionner un clone fournissant en éloctrophorèse les bandes attendues.

Le plasmide purifié comprend la séquence nucléotidique d'origine murine attendue complémentaire de la partie C absente de la séquence de chaîne de mammifère ci-dessus à partir du même site de restriction de manière à permettre la ligation en phase dans le cadre lecture.

De ce qui précède, on comprend que le procédé de la présente invention permet une préparation facile de panels d'anticorps, notamment monoclonaux, spécifiques des différentes structures de la partie V des chaînes du TCR, à partir d'un nombre très limité de segments d'origine murine.

En effet, ledit segment murin codant pour la partie C comprend par modification un site de restriction qui est présent sur la quasi totalité des chaînes constantes d'origine humaine, par exemple BglII pour les chaînes humaines Cβ1 et Cβ2.

Les différentes techniques utilisées ci-dessus sont bien connues de l'homme de l'art et ont fait l'objet de nombreuses publications faisant partie de l'état de la technique.

Les exemples qui suivent illustrent la présente invention sans toutefois la limiter.
La Figure 1 représente les comparaisons des ADNc de chaînes Cβ2 de souris et Cβ1 humaines de TCR.
La Figure 2 représente un oligonucléotide complémentaire de l'ADNc codant pour la chaîne Cβ2 du TCR murin sauf au niveau des deux bases nécessaires pour introduire le site BglII.
La figure 3 représente des oligonucléotides spécifiques de chaînes Cβ1 et Cβ2 de TCR humains.
La figure 4 représente les profils de cytométrie de flux de transfectants de DOIS19 (profils noirs) obtenus avec deux ADN chimériques contenant Vβ8 humain (cadres du haut : gauche FRN8.1.2, droite MZ3 DC8) et Vβ2 humains (cadres du bas : gauche FRN2.7.3, droite FRN2.7.6) ; par rapport à la lignée de départ DOIS19 (profils blancs) ; les cellules sont marquées avec un anticorps anti CD3 de souris.
La figure 5 montre l'analyse de la lignée Jurkatt par un anticorps distinct, le surnageant de E83C11.20 et le monoclonal anti CD3 humain d'Immunotech.

### PARTIE EXPERIMENTALE

### EXEMPLE 1

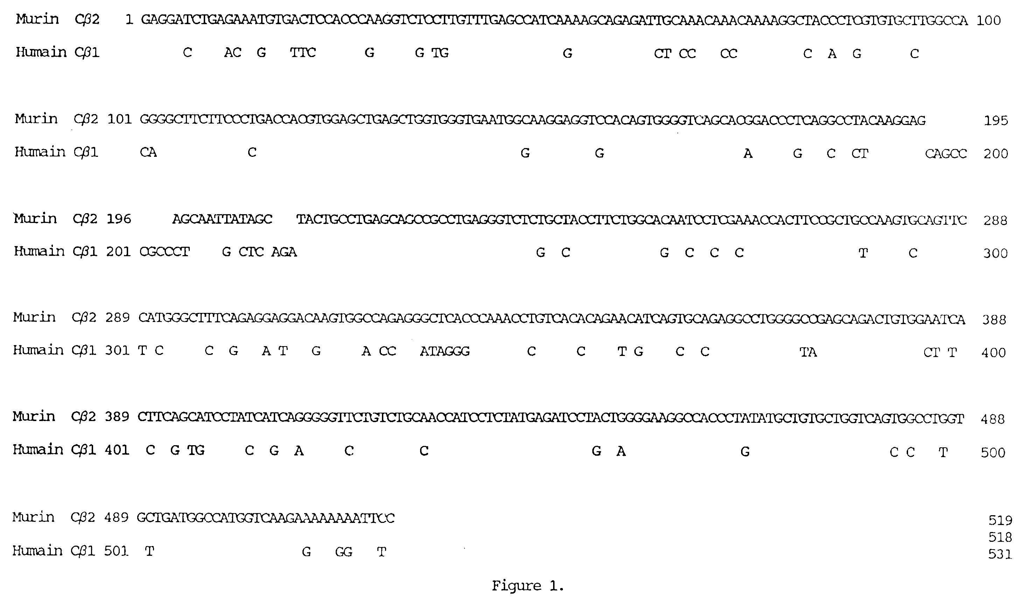

On tire parti de l'existence d'un site BglII sur le gène codant pour les chaines β1 et β2 du TCR dans leur région C, à proximité de la région J, et de celle d'un site identique à l'exception de deux bases dans la partie homologue de la souris (cf. Figure 1).

### A) Préparation de la séquence nucléotidique d'origine murine comprenant la partie complémentaire de la partie C absente de la séquence humaine d'une chaîne β2, sous forme d'un vecteur comportant ladite séquence.

### 1. Chaîne Cβ2 murine

La séquence de la chaîne Cβ2 murine est décrite en détail dans Malissen M. et al (1984), Cell 37 : 1101-1110. Un ADNc complet contenant toute la partie codante de Cβ2 a été obtenu à partir d'une banque d'ADNc de la lignée cellulaire murine KB5C20 (décrite dans Albert et al (1982), Immunogenétics 16 : 533-549). Cet ADNc a été cloné dans le plasmide pUC19. Le plasmide résultant pUC m Cβ2 a été transfecté dans la souche bactérienne DH1. La bactérie recombinante a été déposée auprès de la Collection Nationale de Cultures de Microorganismes (CNCM) à Paris le 12 Février 1991 sous le n° I-1032 et disponible sans restriction.

### 2. Mutagénèse dirigée de l'ADNc codant la chaîne Cβ2 murine.

Principe : La procédure de mutagénèse dirigée choisie utilise le phage filamenteux M13mp18 BIORAD. Ce bactériophage est décrit précisément dans Norrander et al (1983) Gene 26 : 101-106. Il est disponible commercialement chez plusieurs fabricants, notamment BIORAD. Le principe de la manipulation est le suivant :
on clone le fragment d'ADN à modifier dans le site de liaisons multiples du phage. Ce vecteur phagique existe sous deux formes au cours de son cycle de vie. La forme réplicative, dans la bactérie, est essentiellement double brin, la forme encapsidée est simple brin. L'ADN des deux formes est purifiable. Un oligonucléotide complémentaire de la région à muter, sauf pour la mutation désirée, est hybridé avec la forme simple brin du phage. Le brin complémentaire est ensuite synthétisé in vitro par une ADN polymérase, utilisant l'oligonucléotide comportant la mutation souhaitée pour initier la réaction. Une ligase est utilisée pour fermer le nouveau brin sur lui- même, à l'extrémité 5' de l'oligonucléotide. Le double brin complètement homologue, sauf pour la mutation voulue, est transfecté dans E. Coli résultant en deux classes de colonies mutantes et non mutantes. Les colonies mutantes sont ensuite sélectionnées.

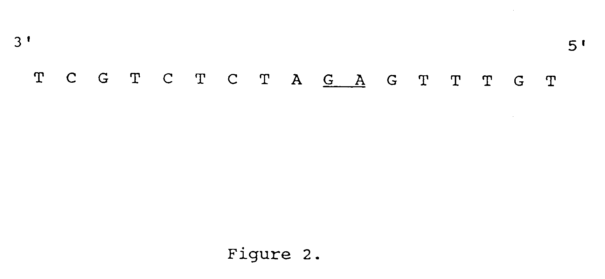

Pour introduire le site BglII dans l'ADNc de la chaîne Cβ2 de souris, on clone cet ADNc dans le phage M13mp18. On utilise l'oligonucléotide de la figure 2 pour faire la mutagénèse. Cet oligonucléotide est complémentaire de l'ADNc codant pour le gène Cβ2 murin sauf pour les deux bases nécessaires pour introduire le site BglII. Ces deux bases sont soulignées sur la figure 2.

Les procédures de clonage, transfection, étalement, analyses des colonies, purification des formes simples et doubles brins pour le phage M13mp18 sont décrites précisément par Maniatis et al (déjà cité). L'hôte bactérien utilisé est la souche JM101 de E. coli.

### Expérimentation :

### a) Clonage de Cβ2 murin dans M13mp18

On digère pUCmCβ2 par Eco RI et Hind III. Cette digestion fournit 3 fragments distinguables par électrophorèse d'agarose. On purifie le fragment intermédiaire de 1000 paires de bases (pb) environ contenant toute la séquence codante de Cβ2 murine. Ce fragment est ligué dans l'ADN double brin du phage M13mp18 préalablement ouvert par Eco RI et Hind III, sites présents dans le site de liaisons multiples de ce vecteur. On transfecte alors des colonies de E. coli JM 101.

De l'analyse de colonies isolées, on retient le clone M13 EH Cβ7 pour la suite, on purifie la forme simple brin de M13EHCβ7.

### b) Mutagénèse de Cβ2 de souris

La procédure utilisée est précisément décrite dans Maniatis et al. On doit cependant préciser les points suivants :
L'oligonucléotide de la figure 2 est synthétisé sur l'appareil Applied Biosystems 380B, puis purifié sur colonne sep Pak C18.
On hybride 0.2 µg du simple brin de M13EHCβ 7 avec 3 pmole de OhCβ kinase. Aucun autre oligonucléotide n'est utilisé. La température initiale de la réaction d'hybridation est 70° C.
La polymérase utilisée est la T4ADN polymérase (1 unité par réaction) pour compléter en double brin.
La transfection est effectuée dans la souche E. coli JM101.
La sélection des colonies mutantes se fait par hybridation avec l'oligonucléotide de la figure 2 radiomarqué. La température d'hybridation est 42° C. Deux lavages de 10 mn à 4° C ont été suffisants pour discriminer les colonies mutantes par autoradiographie.
L'ADN d'un sous-clone appelé muCβ215 d'une colonie repérée par hybridation radioactive a été digérée par BglII, puis séquencé par la méthode de Sanger pour confirmation de la mutation désirée et intégrité du reste de la séquence Cβ2 murine. L'ADN double brin de ce sous-clone est purifié et utilisé dans la suite des expériences.

### 3. Sous-clonage de la chaîne mutée dans p Bluescript SK⁺

p Bluescript SK⁺ est un plasmide entièrement caractérisé et disponible commercialement chez la société STRATAGENE.

L'ADN double brin de muCβ215 est coupé par EcoRI et EcoR V et le plus petit des deux fragments purifié sur gel d'Agarose. Ce fragment est ligué dans p Bluescript préalablement ouvert par EcoRI et Sma I, deux sites présents dans le polylinker de ce plasmide et transfecté dans la souche d'E. coli DH1 servant d'hôte à la chimère. Des colonies isolées sont analysées par coupure avec deux couples d'enzymes EcoRI-XbaI et EcoRI-BglII. Un clone, appelé pBS mu Cβ215, fournit les bandes attendues (respectivement 1.2 kb et 0.5 kb environ) en électrophorèse.

Ce plasmide est purifié et utilisé dans la suite des expériences.

### B) Obtention d'ADNc humain codant pour la chaîne du TCR

La technique de polymérisation en chaîne (PCR) est choisie pour les raisons citées ci-dessous :
- beaucoup de gènes codant pour la chaîne du TCR incluant différentes régions V sont publiées,
- rapidité d'obtention d'ADNc par cette technique,
- sûreté d'avoir des gènes complets en 5', comprenant le codon ATG d'initiation,
- facilité d'intégrer des sites de restriction en 5' du gène pour faciliter le clonage.

Dans l'exemple préféré, et pour montrer la polyvalence permise par l'invention, plusieurs ADNc humains contenant différents segments variables ont été isolés et couplés à la chaîne mutée Cβ2 de souris.

### - Obtention d'ARNm de cellules T humaines

On utilise un mélange de lymphocytes périphériques d'individus caucasiens obtenus auprès du Centre de Transfusion Sanguine de Marseille. Après Ficoll, les lymphocytes sont ajustés à 1 million de cellules par millilitre en milieu RPMI, 10 % sérum de veau foetal 20 mM glutamine, 1mM sodium pyruvate, 500 IU pénicilline, et stimulés à la phytohémagglutinine A (10 µg/millilitre de concentration finale) pendant 3 jours. 200 millions de cellules sont utilisées pour purifier leur RNA total.

Le protocole utilisé est décrit exactement dans Maniatis et al. Environ 50 µg de RNA sont obtenus par cette méthode.

### - Réactions de PCR

Un oligonucléotide OhCβ spécifique des deux chaînes humaines Cβ2 et Cβ1 et contenant le site unique BglII présent sur les segments géniques Cβ1 et Cβ2 est synthétisé de manière analogue à OmCβ.

Cinq oligonucléotides OL1, OL2, OL3, OL8, OL12 spécifiques respectivement de la séquence de tête de Vβ1.2, Vβ2.1, Vβ3.1, Vβ8.1, Vβ12.3 d'après la définition des sous-familles Vβ de Toyonaga et al (1987), Eur J Immunol ; 17 : 375-383 sont également synthétisés.

Ces oligonucléotides possèdent en 5' les 8 bases TAGAATTC n'hybridant pas sur les séquences de têtes des régions variables . Ces 8 bases contiennent le site EcoRI pour faciliter le clonage des produits d'amplification.

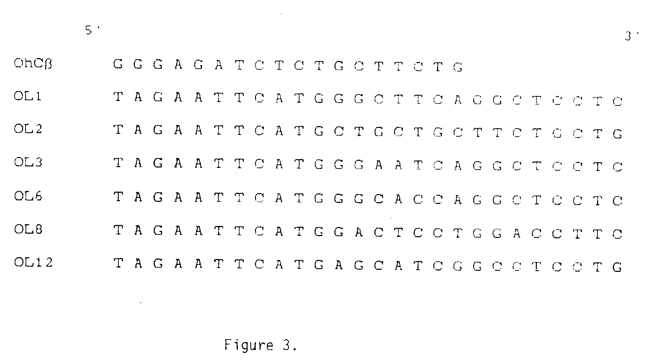

Ces oligonucléotides sont présentés en figure 3.

Cinq réactions de PCR indépendantes ont été faites en utilisant les couples d'oligonucléotides OhCβ-OLi où i est un élément de (1, 2, 3, 8, 12).

Nous avons commencé avec le couple OhCβ, OL8. La procédure expérimentale est décrite en détail dans Kawasaki et al (1989) PCR Technology, Principles and Applications for ADN Amplification ; Stockton Press, Henry. A Ehrlich Edition pp89-et suivantes. Les précisions suivantes sont utiles :
La quantité d'ARN de départ est 1 µg pour la synthèse du brin complémentaire d'ADNc.
Pour la réaction de PCR proprement dite, 10 pmole de chaque oligonucléotide OL8 et OhCβ, sont utilisés.

Le cycle de PCR est :
- 1 mn de dénaturation à 95° C,
- 1 mn d'hybridation à 52° C,
- 1 mn d'extension à 72° C,
et 30 cycles sont effectués sur le ADN Thermalcycler Version 2.2 de Perkin Elmer.

Le produit de la réaction est déposé sur gel d'Agarose 1.4 % pour électrophorèse. Après coloration au bromure d'éthidium, une bande distincte d'environ 500 pb est découpée et l'ADN électroélué. On obtient environ 30 ng d' ADN.

1 ng de cet ADN est amplifié une seconde fois par PCR. On purifie de la même manière que précédemment la bande spécifique et 300 ng d'ADN sont obtenus.

Les réactions de PCR avec les couples OL1, OL2, OL3, OL12 associés respectivement à OhCβ sont effectués de la même manière, sauf que dans le cas de OL12 et OL3, la deuxième amplification n'a pas été nécessaire (on a obtenu plus de 100 ng en première amplification).

### C) Construction des gènes chimériques

### Principe :

On construit les gènes chimériques en clonant chaque fragment produit de PCR, coupé par EcoRI et BglII dans le grand fragment obtenu de la digestion de pBSmuCβ215.

On coupe pBSmuCβ215 par EcoRI et BglII et le grand fragment appelé FpBSmuCβ215 est purifié sur gel d'Agarose et électroélué.

100 ng du produit d'amplification obtenu avec les oligonucléotides OL8, OhCβ sont coupés par EcoRI et BglII.

On ligue ces deux fragments et le produit de ligation est transfecté dans DH1.

L'analyse de colonies isolées BSGβ8.1, BSGβ8.2 ... BSGβ8.5 par coupure BglII-EcoRI fournit le fragment attendu d'environ 500 pb. BSG 8.1 est séquencé entre le site EcoRI et le site BgIII. L'analyse de la séquence montre une similarité totale avec la séquence publiée pour Vβ8.1 de Yoshikai Y et al (1984) Nature 312 pp521-524.

De même, on clone les produits de PCR obtenus avec les couples d'oligonucléotides contenant OL1, OL2, OL3, OL12 dans FpBSCβ215.

On obtient par rapport à la définition des différentes sous-familles Vβ par Toyonaga et al (1987) Eur J Immunol, 17 : 375-383 :
. avec OL1, les clones BSGβ1.2 et BSGβ1.3 dont la séquence pour la région V est identique à Vβ1.2.
. avec OL2, les clones BSGβ2.5 dont la séquence pour la région V correspond à Vβ2.2 et BSGβ2.7 et BSGβ2.11 possédant le gène Vβ2.3.
. avec OL12, les clones BSGβ12.1 et BSGβ12.4 possédant le gène Vβ12.3.

### EXEMPLE 2 : Expression des gènes chimériques

### Principe :

La chaîne du TCR est une protéine membranaire qui est exprimée à la surface si, et seulement si, les autres chaînes du complexe du TCR qui lui sont associées (ie chaîne α et complexe CD3) sont présentes.

Pour obtenir l'expression des gènes chimériques précédemment construits, on utilise la lignée cellulaire murine DOIS19 développée par Letourneur F et al (1989) Eur J Immunol : 19 : 2269-2274. DOIS19 est un mutant d'un hybridome T de souris qui possède tous les composants du TCR sauf la chaîne β. Cette lignée n'exprime donc pas de TCR à sa surface. La transfection d'une chaîne exogène induit l'expression d'un récepteur fonctionnel dans les cellules tranfectées. Deux gènes chimériques de l'invention contenus dans BSGβ8.1 et BSGβ2.7 ont été transfectés dans cette cellule suivant les protocoles décrits ci-après :

### 1) Sous-clonage des gènes chimériques dans un vecteur d'expression eucaryote

### Principe :

Le vecteur d'expression choisi est le plasmide pHβPr 1-néo. Il est décrit précisément dans Gunning P et al (1987) Proc Natl Acad Sci USA ; 84 : 4831-4835. Le gène à exprimer est sous-cloné en aval du promoteur β actine et en amont d'un site de polyadénylation présents sur ce plasmide. Ces deux signaux assurent la synthèse d'un RNA messager fonctionnel du gène inséré. Par ailleurs, ce plasmide possède le gène de résistance néomycine qui confère la résistance à une série d'antibiotiques, dans l'exemple préféré l'antibiotique G418, aux cellules ayant intégré le plasmide.

Par ailleurs, une origine de réplication et un gène de résistance à l'ampicilline sont également présents permettant la sélection et la réplication dans E coli. La souche de E. coli utilisée est ici MC 1061.

### Expérimentation :

On coupe le plasmide BSGβ8.1 par les enzymes Bam HI et Sal I et le plus petit des deux fragments (1.2 kb) est purifié sur gel d'Agarose. Ce fragment comportant la totalité de la séquence codante du gène chimérique est ligué au plasmide pHβ Prl-néo préalablement ouvert par Bam HI et Sal I. Le produit de ligation est transfecté dans E. coli MC 1061 et de l'analyse de colonies isolées par la coupure Bam HI et Sal I, on retient le clone appelé néo Gβ8.1 qui fournit le fragment de 1.2 kb attendu sur l'électrophorèse d'Agarose.

De la même façon, le gène chimérique contenu dans pBSGβ2.7 est sous-cloné dans pHβPr1-néo et le clone appelé néo Gβ2.7 est conservé. Néo Gβ8.1 a été déposé à la CNCM le 12 février 1991 sous le n° I-1033.

### 2. Transfection des gènes chimériques dans DOIS 19

La procédure de transfection choisie est la fusion de protoplastes.

On suit pas à pas le protocole décrit par MALISSEN et al (réf. Technique de transfert de gènes dans les cellules eucaryotes" 1990, Editions INSERM. Paris, 25-31) sauf que la concentration de G418 dans le milieu sélectif est de 3 mg/ml avec les clones néo Gβ8.1 et néo Gβ2.7.

### 3. Analyses des transfectants

Pour néo Gβ8.1, les colonies résistantes à la néomycine appelées FRN8.1.1 ... FRN8.1.24 sont analysées en cytométrie de flux. Pour chaque clone, 10⁵ cellules dans une solution de Dubelco Modified Eagle Medium (DMEM) à 4 % de sérum de veau foetal (SVF), 0.02 % Azide de sodium et 15 µg/ml d'anticorps anti CD3 de souris (anticorps 2C11 décrits dans LEO et al (1987) Proc Natl Acad Sci USA ; 84 : 1374) pendant 45 mn à 4° C sous agitation. Après trois lavages en DMEM, 4 % SVF, 0.02 % Azide de sodium, on incube les cellules avec un anticorps polyclonal de chèvre anti immunoglobuline de rat marqué au FITC (réactif Immunotech), à 15 µg/ml en DMEM, 4 % SVF, 0.02 % azide pendant 30 mn à 4° C sous agitation. Après trois lavages en DMEM, 4 % SVF, 0.02 % azide, les cellules sont fixées en PBS à 1 % formaldéhyde et analysées sur l'appareil de cytométrie de flux FACSCAN de Becton Dickinson. 10⁵ cellules DOIS 19 sont traitées de la même façon et servent de contrôle négatif.

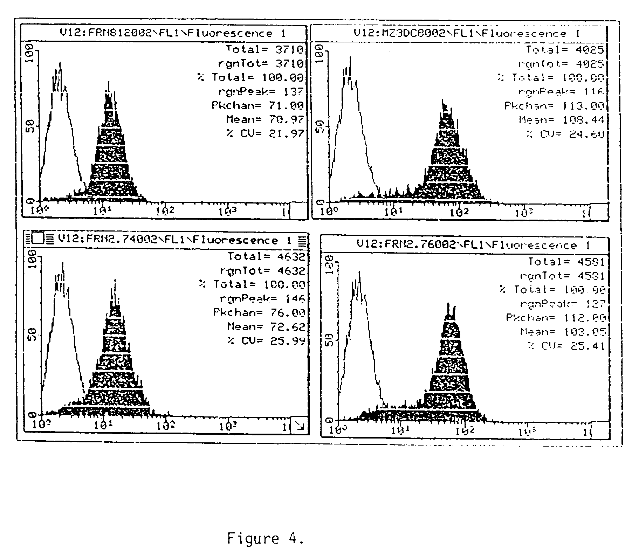

Le profil obtenu pour le clone FRN8.1.2 est montré en figure 4 par rapport au profil de DOIS 19.

On procède de même avec le plasmide néo Gβ2.7 et le profil obtenu sur FACSCAN du clone FRN2.7.3 est montré sur la fig 4 par rapport à DOIS 19. Les deux lignées cellulaires sont retenues pour l'immunisation de souris Balb/c.

### 4. Immunisation des souris Balb/c

Trois souris Balb/c sont immunisées avec FRN8.1.2. Le protocole d'immunisation est le suivant :
- Jour 0 : 10 millions de cellules FRN8.1.2 injectées par voie intrapéritonéale.
- Jour 20 : 10 millions de cellules FRN8.1.2 injectées par voie intrapéritonéale.
- Jour 40 : 5 millions de cellules injectées par voie intraveineuse.
- Jour 43 : Prélèvement des rates pour fusion.

### 5. Fusion par hybridation lymphocytaire

On fusionne les splénocytes de deux souris immunisées avec le myélome X63 Ag 258 suivant le protocole décrit par Kohler et Milstein (1975) Nature 256 ; 495-497.

L'analyse des surnageants des clones résultant de la fusion se fait par cytométrie de flux sur FRN8.1.2 versus DOIS 19. 100 µl de surnageant de chaque clone est prélevé, 50 µl sont incubés avec 50 µl de DMEM, 4 % SVF, 0.02 % azide contenant 10⁵ cellules FRN8.1.2, les 50 µl restants sont incubés avec 50 µl du même milieu mais contenant 10⁵ cellules DOIS 19. Le protocole est le même par ailleurs que celui utilisé pour l'analyse des transfectants.

Des 400 surnageants testés, deux surnageants marquent FRN8.1.2 et ne marquent pas DOIS19. Les deux monoclonaux correspondant réagissent donc avec une chaîne du complexe chimérique du TcR contenant la chaîne humaine Vβ8.1.

Le clone E83C11.20 ne marque pas la lignée FRN2.7.3 assurant que ce monoclonal ne reconnaît pas les composants souris du TcR chimérique.

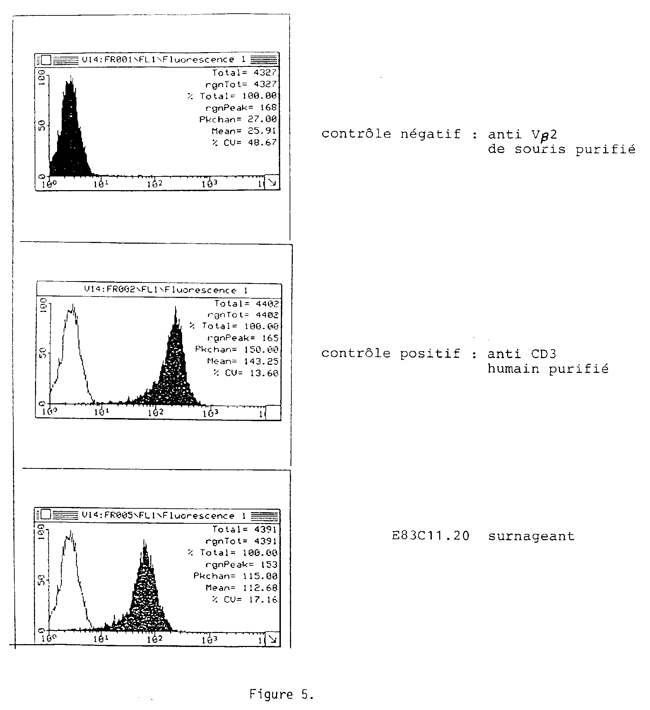

Le clone E93C11.20 marque par contre la lignée humaine Jurkatt exprimant la chaîne Vβ8.1 (Yoshikai et al (1984) Nature : 312 : 521-524). La fig. 5 montre l'analyse de la lignée Jurkatt par un anticorps distinct, le surnageant de E83C11.20 et le monoclonal anti CD3 humain d'Immunotech.

De ces analyses, on conclut que le monoclonal E83C11.20 est spécifique d'une chaîne humaine Vβ8.1.

### EXEMPLE 3

On a préparé une préparation injectable comprenant :
- Anticorps monoclonaux de l'exemple 2 5 mg
- eau pour préparations injectables 1 ml

## Revendications

1. ADN recombinant caractérisé en ce qu'il comprend une séquence nucléotidique chimère codant pour une chaîne du récepteur pour l'antigène des cellules T, ladite séquence étant constituée :
- d'une séquence provenant d'un mammifère autre que murin et codant pour la totalité de la partie variable (V), diversité (D) et de jonction (J),
- et d'une séquence d'origine murine codant pour la partie complémentaire constante (C) absente de la séquence de mammifère ci-dessus,
les deux séquences étant liées au niveau d'un site de restriction naturel ou créé artificiellement.

2. ADN recombinant selon la revendication 1 caractérisé en ce que le site de restriction est naturel sur la séquence codant pour la partie C.

3. ADN recombinant selon la revendication 1 ou 2 caractérisé en ce que la séquence provenant d'un mammifère est la séquence codant pour une chaîne Vβ d'origine humaine et en ce que le site de restriction est BglII.

4. ADN recombinant selon l'une quelconque des revendications 1 à 3 caractérisé en ce que le site de restriction est créé artificiellement sur la chaîne d'origine murine.

5. ADN recombinant selon l'une quelconque des revendications 1 à 4 caractérisé en ce qu'il s'agit d'un vecteur d'expression eucaryote.

6. ADN recombinant selon la revendication 5 caractérisé en ce que le vecteur d'expression eucaryote est un plasmide dérivant de Phβ -Pr1-Néo.

7. ADN recombinant selon l'une quelconque des revendications 1 à 6 caractérisé en ce que la partie variable (V) est d'origine humaine et choisie parmi Vβ1, Vβ2 Vβ3, Vβ4, Vβ5, Vβ6, Vβ7, Vβ8, Vβ10, Vβ11, Vβ12, Vβ15, Vβ17 et Vβ18.

8. ADN recombinant selon la revendication 7 caractérisé en ce que la partie variable (V) est Vβ2.

9. ADN recombinant selon la revendication 7 caractérisé en ce que la partie variable (V) est Vβ17.

10. Procédé de préparation d'anticorps comprenant l'expression d'hybridomes préparés par fusion lymphocytaire à partir de cellules de rates de souris immunisées à l'aide d'un antigène contenant une protéine caractérisé en ce que ladite protéine est codée par un ADN recombinant tel que défini à l'une quelconque des revendications 1 à 9.

11. Compositions pharmaceutiques caractérisées en ce qu'elles renferment des anticorps préparés selon le procédé de la revendication 10.

12. Produits de diagnostic caractérisés en ce qu'ils renferment des anticorps préparés selon le procédé de la revendication 10.

13. Procédé de préparation d'un ADN recombinant tel que défini à la revendication 1, caractérisé en ce que l'on ligue :
- une séquence nucléotidique constituée d'au moins la totalité de la séquence codant pour les parties V, D et J d'une chaîne du récepteur de l'antigène des cellules T d'un mammifère autre que murin jusqu'au niveau d'un site de restriction, avec
- une séquence nucléotidique d'origine murine comprenant la partie complémentaire de la partie C absente de la séquence de chaîne de mammifère ci-dessus, à partir du même site de restriction que ci-dessus,
ledit site de restriction étant artificiellement créé sur l'une, l'autre ou les deux séquences, pour obtenir la chimère attendue, puis si désiré, ligue une séquence comprenant la totalité des parties V,D,J et C ci-dessus avec un vecteur d'expression eucaryote, puis si désiré, isole la nouvelle chimère obtenue.

14. Procédé de préparation d'anticorps selon la revendication 10 qui comporte une étape de préparation de l'ADN recombinant défini à la revendication 5 caractérisé en ce qu'en outre l'on transfecte une cellule eucaryote comportant tous les éléments permettant la synthèse du récepteur de l'antigène des cellules T, à l'exception de la chaîne homologue à la chaîne codée par la séquence d'ADN chimère ci-dessus, utilise comme antigène les cellules ainsi transfectées chez la race murine correspondante, fusionne des cellules de rate prélevées sur les murins auxquels a été administré l'antigène à des cellules de myélome pour préparer, selon les méthodes connues en elles-mêmes, des anticorps dirigés contre le produit de la séquence variable du mammifère.

15. Anticorps anti-chaîne Vβ2 du récepteur des cellules T humaines.

16. Anticorps anti-chaîne Vβ17 du récepteur des cellules T humaines.

## Claims

1. Recombinant DNA characterized in that it contains a chimaeric nucleotide sequence coding for T-cell antigen receptor chains, said sequence being constituted:
- by a sequence derived from a mammal other than murine and coding for all the variable part (V), diversity part (D) and junction part (J),
- and by a sequence of murine origin coding for the constant complementary part (C) absent from the mammal sequence above,
the two sequences being linked at the level of a natural or artificially created restriction site.

2. Recombinant DNA according to claim 1, characterized in that the restriction site is natural on the sequence coding for the C part.

3. Recombinant DNA according to claim 1 or 2, characterized in that the sequence deriving from a mammal is the sequence coding for a Vβ chain of human origin and in that the restriction site is BglII.

4. Recombinant DNA according to any one of claims 1 to 3, characterized in that the restriction site is created artificially on the chain of murine origin.

5. Recombinant DNA according to any one of claims 1 to 4, characterized in that it is a eukaryotic expression vector.

6. Recombinant DNA according to claim 5, characterized in that the eukaryotic expression vector is a plasmid deriving from Phβ -Pr1-Neo.

7. Recombinant DNA according to any one of claims 1 to 6, characterized in that the variable part (V) is of human origin and is chosen from Vβ1, Vβ2, Vβ3, Vβ4, Vβ5, Vβ6, Vβ7, Vβ8, Vβ10, Vβ11, Vβ12, Vβ15, Vβ17, and Vβ18.

8. Recombinant DNA according to claim 7, characterized in that the variable part (V) is Vβ2.

9. Recombinant DNA according to claim 7, characterized in that the variable part (V) is Vβ17.

10. Preparation process for an antibody comprising the expression of hybridomas prepared by lymphocytic fusion starting from the spleen cells of mice immunized with an antigen containing a protein characterized in that said protein is coded by a recombinant DNA as defined in any one of claims 1 to 9.

11. Pharmaceutical compositions, characterized in that they contain antibodies prepared according to the process of claim 10.

12. Diagnostic products, characterized in that they contain antibodies prepared according to the process of claim 10.

13. Preparation process for a recombinant DNA as defined in claim 1, characterized in that the following are ligated:
- a nucleotide sequence constituted by at least all of the sequence coding for the V,D and J parts of a chain for a T-cell antigen receptor of a mammal other than murine up to the level of a restriction site, with
- a nucleotide sequence of murine origin comprising the complementary part of the C part absent from the mammalian chain sequence above, starting from the same restriction site as above,
said restriction site being artificially created on one, the other or both sequences, in order to obtain the expected chimaera, then if desired, a sequence containing all of parts V, D, J and C above is ligated with a eukaryotic expression vector, then if desired, the new chimaera obtained is isolated.

14. Preparation process for an antibody according to claim 10 which comprises a preparation stage for a recombinant DNA defined in claim 5, characterized in that in addition a eukaryotic cell containing all the elements allowing the synthesis of a T-cell antigen receptor, with the exception of the chain homologous to the chain coded by the chimaeric DNA sequence above is transfected, the cells thus transfected are used as antigen for the corresponding murine breed, the spleen cells taken from the murines to which the antigen has been administered are fused to the myeloma cells in order to prepare, according to the methods known per se, antibodies directed towards the product of the variable sequence of the mammals.

15. Vβ2 anti-chain antibody for human T-cell receptors.

16. Vβ17 anti-chain antibody for human T-cell receptors.

## Patentansprüche

1. Rekombinante DNS, dadurch gekennzeichnet, daß sie eine chimäre Nucleotidsequenz umfaßt, die für eine Kette des Rezeptors für das T-Zellen-Antigen codiert, wobei die Sequenz besteht aus:
- einer Sequenz, die von einem anderen als murinen Säuger stammt und für die Gesamtheit des variablen (V), Diversitäts-(D) und Vereinigungsteils (J) codiert,
- und einer Sequenz murinen Ursprungs, die für den konstanten komplementären Teil (C) codiert, der in der obigen Säugersequenz fehlt,
wobei die beiden Sequenzen auf der Höhe einer natürlichen oder künstlich geschaffenen Restriktionsstelle verbunden sind.

2. Rekombinante DNS nach Anspruch 1, dadurch gekennzeichnet, daß die Restriktionsstelle natürlich auf der für den Teil C codierenden Sequenz ist.

3. Rekombinante DNS nach Anspruch 1 oder 2, dadurch gekennzeichnet, daß die von einem Säuger stammende Sequenz die für eine Vβ-Kette menschlichen Ursprungs codierende Sequenz ist, und daß die Restriktionsstelle BglIII ist.

4. Rekombinante DNS nach einem der Ansprüche 1 bis 3, dadurch gekennzeichnet, daß die Restriktionsstelle auf der Kette murinen Ursprungs künstlich geschaffen wird.

5. Rekombinante DNS nach einem der Ansprüche 1 bis 4, dadurch gekennzeichnet, daß es sich um einen Eukaryoten-Expressionsvektor handelt.

6. Rekombinante DNS nach Anspruch 5, dadurch gekennzeichnet, daß der Eukaryoten-Expressionsvektor ein von Phβ-Prl-Neo abgeleitetes Plasmid ist.

7. Rekombinante DNS nach einem der Ansprüche 1 bis 6, dadurch gekennzeichnet, daß der variable Teil (V) menschlichen Ursprungs ist und aus Vβ1, Vβ2, Vβ3, Vβ4, Vβ5, Vβ6, Vβ7, Vβ8, Vβ10, Vβ11, Vβ12, Vβ15, Vβ17 und Vβ18 ausgewählt wird.

8. Rekombinante DNS nach Anspruch 7, dadurch gekennzeichnet, daß der variable Teil (V) Vβ2 ist.

9. Rekombinante DNS nach Anspruch 7, dadurch gekennzeichnet, daß der variable Teil (V) Vβ17 ist.

10. Verfahren zur Herstellung eines Antikörpers, welches die Expression von Hybridomen umfaßt, die durch Lymphocytenfusion aus Milzzellen von Mäusen hergestellt werden, welche mit Hilfe eines Antigens immunisiert wurden, das ein Protein enthält, dadurch gekennzeichnet, daß eine rekombinante DNS wie in einem der Ansprüche 1 bis 9 definiert für das Protein codiert.

11. Pharmazeutische Zusammensetzungen, dadurch gekennzeichnet, daß sie gemäß dem Verfahren von Anspruch 10 hergestellte Antikörper enthalten.

12. Diagnoseprodukte, dadurch gekennzeichnet, daß sie gemäß dem Verfahren von Anspruch 10 hergestellte Antikörper enthalten.

13. Verfahren zur Herstellung einer rekombinanten DNS wie in Anspruch 1 definiert, dadurch gekennzeichnet, daß ligiert wird:
- eine Nucleotidsequenz, die aus zumindest der Gesamtheit der für die Teile V, D und J codierenden Sequenz einer Kette des Rezeptors des T-Zell-Antigens eines anderen als murinen Säugers bis zur Höhe einer Restriktionsstelle besteht, mit
- einer Nucleotidsequenz murinen Ursprungs, die den komplementären Teil des Teils C, der in der Sequenz der obigen Säugerkette fehlt, aus derselben Restriktionsstelle wie oben umfaßt,
wobei die Restriktionsstelle künstlich auf einer, der anderen oder den beiden Sequenzen geschaffen wird, um die erwartete Chimäre zu erhalten, dann, wenn gewünscht, eine Sequenz, welche die Gesamtheit der obigen Teile V, D, J und C umfaßt, mit einem Eukaryoten-Expressionsvektor ligiert wird, und anschließend, wenn gewünscht, die neue erhaltene Chimäre isoliert wird.

14. Verfahren zur Herstellung eines Antikörpers nach Anspruch 10, welches einen Schritt der Herstellung von wie in Anspruch 5 definierter rekombinanter DNS umfaßt, dadurch gekennzeichnet, daß außerdem eine Eukaryotenzelle transfektiert wird, die alle Elemente umfaßt, welche die Synthese des Rezeptors des T-Zellen-Antigens ermöglichen, mit Ausnahme der Kette, die zu der Kette homolog ist, für welche die obige chimäre DNS-Sequenz codiert, die so transfektierten Zellen in der entsprechenden murinen Rasse als Antigen verwendet werden, die Milzzellen, die den Murinen entnommen wurden, denen das Antigen für die Myelom-Zellen verabreicht wurde, fusioniert werden, um gemäß an sich bekannten Verfahren Antikörper herzustellen, die gegen das Produkt der variablen Säugersequenz gerichtet sind.

15. Antikörper gegen die Vβ2-Kette des humanen T-Zellen-Rezeptors.

16. Antikörper gegen die Vβ17-Kette des humanen T-Zellen-Rezeptors.
